# EUROPEAN PATENT APPLICATION

(11) **EP 1 186 313 A1**
(43) Date of publication of application: **13.03.2002**
(21) Application number: 00203047.6
(22) Date of filing: 05.09.2000
(51) Int. Cl.: A61M 5/32

(54) **Safety cover for a hypodermic syringe**

(71) Applicant: Chen, Chien-Liang, Nantun Dist., Taichung (TW)
(72) Inventor: Chen, Chien-Liang, Nantun Dist., Taichung (TW)
(74) Representative: De Hoop, Eric

(57) **Abstract**

A safety cover for a syringe includes a tubular body (10) containing a receiving space (100). The tubular body (10) includes a first end, a second end and a longitudinal slot (11) defined in the tubular body (10) beginning at the first end and extending toward the second end. A passage (12) is defined in the bottom of the slot (11) to communicate with the receiving space (100) in the tubular body (10). The passage (12) allows the needle (21) of syringe to pass through the tubular body (10) and into the receiving space (100) to prevent the needle (21) from accidentally pricking the medical worker by changing the direction the needle (21) moved when installing the cover.

## Description

The present invention relates to a safety cover, and more particularly to a safety cover for the needle of a hypodermic syringe.

A conventional cover for a syringe in accordance with the prior comprises a tubular body having at least one open end to allow the needle to pass into the tubular body and attach to one end of the syringe after an injection. The axis of the cover is moved along the needle to cover the needle. The medical worker may accidentally prick his finger with the needle when covering the needle of the syringe due to carelessness.

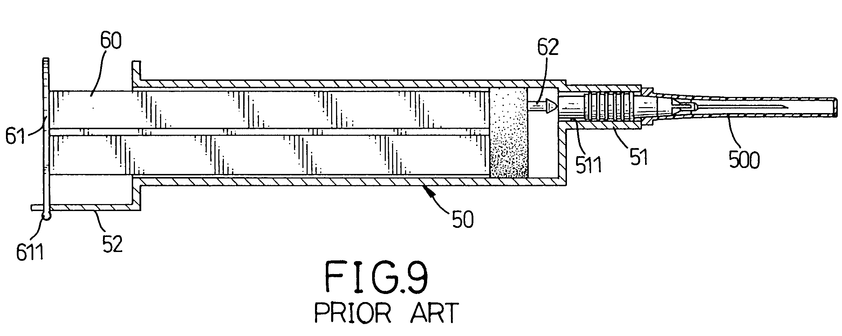

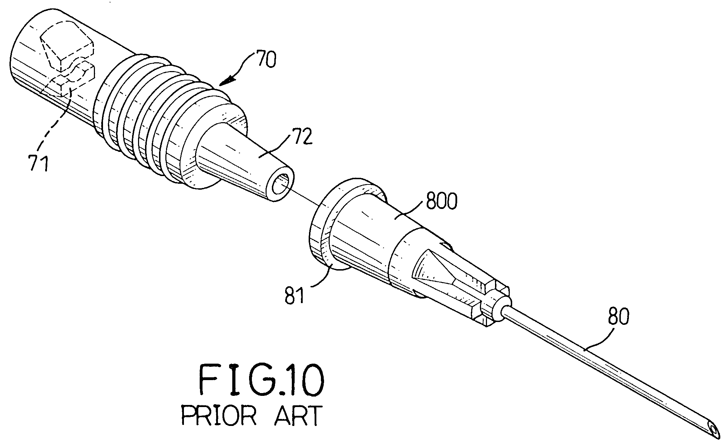

Consequently, a syringe with a retractable needle was developed to prevent unexpectedly pricking and the resultant infection. With reference to Figs. 9-10, the syringe comprises a barrel (50) including a hollow stub (51) extending from a first end and communicating with the inside of the barrel (50) and a flexible positioning plate (52) extending down from a second end. The stub (51) includes a shoulder (511) extending radially inward at the free end of the stub (51). A needle (80) is securely mounted in a needle hub (800) having an annular flange extending radially out from the end of the hub (800). The needle (80) extends through the stub (51) of the barrel (50) and the flange (81) of the needle hub (800) that abuts the shoulder (511) of the stub (51) to prevent the needle (80) from detaching from the barrel (50). A tubular connector (70) is detachably received in the stub (51) of the barrel (50). A tapered connecting rod (72) extends from a first end of the connector (70) to be securely received in the needle hub (800), and a hollow cylindrical rod (not numbered) extends from a second end of the connector (70). At least two jaws (71) extend radially from the internal sidewall of the hollow cylindrical rod. A plunger (60) is slidably received in the barrel (50). A T-shaped connecting bar (62) extends from a first end the plunger (60) to connect to the jaw (71) in the hollow cylindrical rod of the connector (70), and a bottom plate (61) extends radially out from a second end. A protrusion (611) extends radially out from the bottom plate (61) to engage with the positioning plate (52) on the barrel (50) to prevent the connecting bar (62) from being inserted into the hollow cylindrical rod of the connector (70) before the syringe is used.

When the connecting bar (62) on the plunger (60) is inserted into the hollow cylindrical rod and engaged with the jaws (71), the connector (70) and the needle can be pulled back into the barrel (50) to prevent the used needle from pricking a medical worker. However, the structure of the syringe with a retractable needle has several disadvantages as follow.
1. It is hard to assemble. The needle (80) and the connector (70) must be mounted in the limited space defined in the stub (51). It is hard to assemble unless a special tool is used.
2. The price of the syringe with a retractable needle is high. All the parts of the syringe are different from an ordinary syringe except the needle (80). The manufacturer must prepare several unique molds to produce the syringe. The cost of making the syringe is very high.
3. The function of the syringe with a retractable needle is limited. The syringe cannot be used to draw blood because the needle (80) cannot be retracted after drawing blood since the barrel (50) is filled with blood and the connecting bar (62) cannot connect with the jaws (71). Then the conventional cover can not protect the medical worker from unexpectedly pricking and the resultant infection.

The present invention has arisen to mitigate and/or obviate the disadvantages of the conventional syringe.

In accordance with one aspect of the present invention, a safety cover for a syringe is provided to protect medical workers from accidental pricking and the resultant infection. The safety cover comprises a tubular body having a first end, a second end and an outer periphery including a slot defined in the first end and extending towards the second end. The slot includes a bottom having a passage defined to communicate with a receiving of the tubular body and the slot. The passage allows the needle of syringe radially passing therethrough and into the receiving space of the tubular body to prevent the needle from pricking the medical worker by changing the moved direction of the needle after using.

Further benefits and advantages of the present invention will become apparent after a careful reading of the detailed description with appropriate reference to the accompanying drawings. In the drawings:
Fig. 1 is a perspective view of a safety cover for a syringe in accordance with the present invention;
Fig. 2 is a cross-sectional side plan view of the safety cover for the syringe in Fig. 1;
Fig. 3 is an operational cross sectional top view of the safety cover for a syringe in Fig. 2 and a syringe needle prepared to be inserted into the safety cover;
Fig. 4 is an operational cross sectional top view of the safety cover for a syringe along line 4-4 in Fig. 2 and a syringe needle being inserted into the safety;
Fig. 5 is a perspective view of a second embodiment of a safety cover for a syringe in accordance with the present invention;
Fig. 6 is a cross sectional side plan view of the safety cover for a syringe in Fig. 5;
Fig. 7 is an operational cross sectional top plan view of the safety cover for a syringe along line 7-7 in Fig. 6 and a needle hub being attached to a syringe;
Fig. 8 is a cross-sectional cross sectional top plan view of a third embodiment of a safety cover for syringe in accordance with the present invention;
Fig. 9 is a cross-sectional side plan view of a conventional safety syringe in accordance with the prior art; and
Fig. 10 is an exploded perspective view of the conventional retractable needle assembly in Fig. 9.

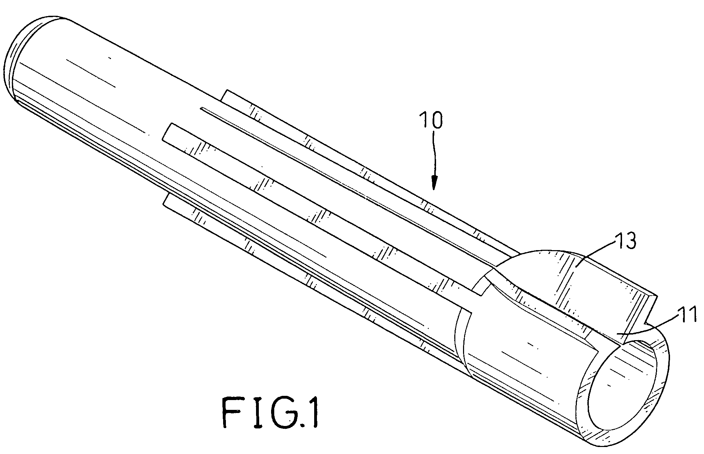

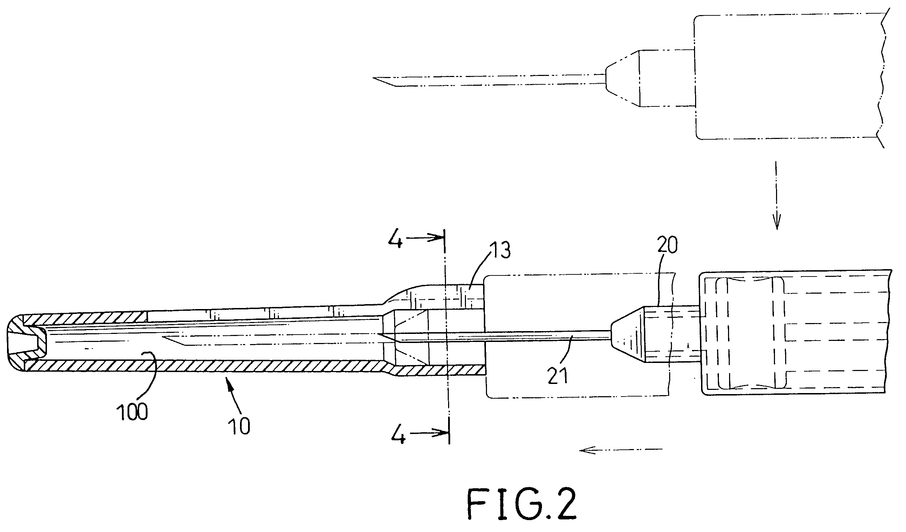

With reference to the drawings and initially to Figs. 1-3, a safety cover for a syringe in accordance with the present invention comprises a tubular body (10) with a first end, a second end and a receiving space (100). The receiving space (100) is configured to receive a needle (21) and a needle hub (20) of a conventional syringe.

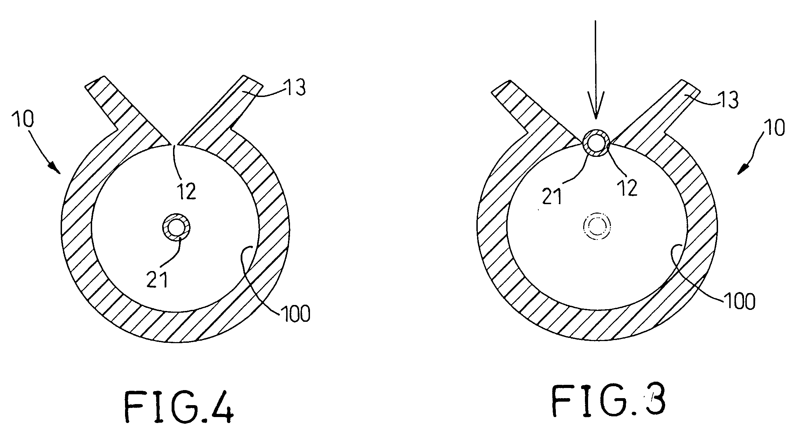

The tubular body (10) includes a longitudinal slot (11) starting at the open end and extending towards the closed end. The length of the slot (11) is substantially longer than an ordinary needle (21). The slot (11) in the tubular body (10) is V-shaped. A passage (12) is formed in the bottom of the slot (11) to communicate with the receiving space (100) in the tubular body (10). The passage (12) allows the needle (21) to pass through the slot (11) to be received into the receiving space (100) in the tubular body (10) without having to thread the cover along the central axis of the needle (21). The width of the passage (12) is slightly smaller than the diameter of the needle (21), which keeps the needle (21) from coming out of the slot (11) once the safety cover has been put over the needle (21). Two wings (13) respectively extend out from tubular body (10) along the angle of the sidewall of the slot (11) to form a funnel-shape. The length of the wing (13) is shorter than the slot (11). With reference to Fig. 4, the two wings (13) guide the needle (21) into the slot (11). Pressing the needle (21) widens the passage (12) to allowing the needle (21) to pass through the slot (11) and the passage (12) into the receiving space (100). With the needle (21) inside the receiving space (100), the user moves the safety cover along the axis of the needle (21) to attach the safety cover to the needle hub (20) on the syringe.

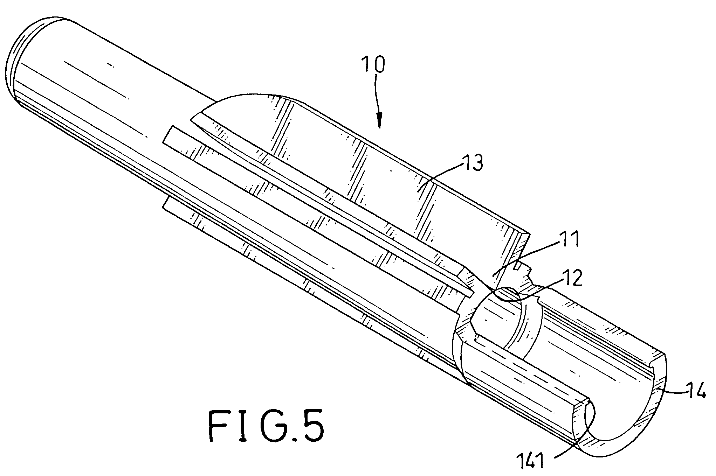

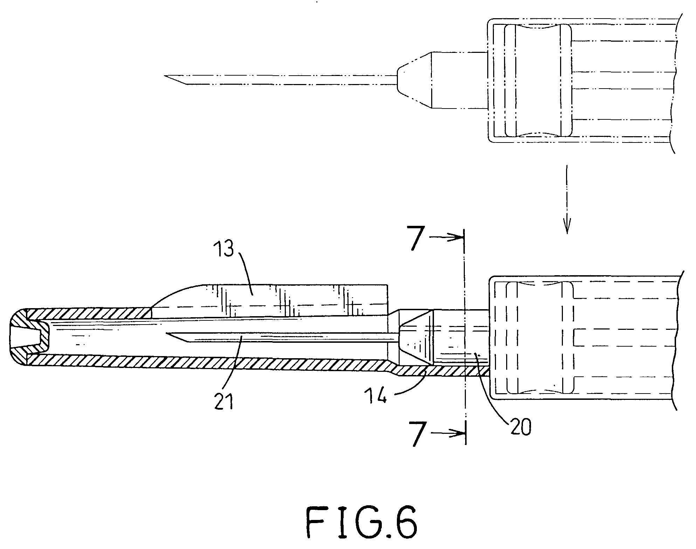

With reference to Fig. 5-7, in a second embodiment of the safety cover for a syringe in accordance with the present invention, a cutout is defined in the open end of the tubular body (10) to form a C-shaped clamp (14). A rib (141) extends inward from each edge of the clamp (14). The distance between the two edges of the clamp (14) is slight smaller than the diameter of the needle hub (20).

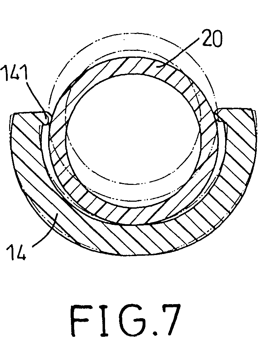

The needle (21) is guided into the bottom of the slot (11) by the two wings (121) as shown in Fig. 3 when attaching the safety cover to the syringe. With reference to Fig. 4 and Fig. 7, the needle (21) widens the passage (12), and the needle hub (20) press the ribs (141) to widen the clamp (14) at the same time to allow the needle to pass into the receiving space (100) and the needle hub (20) to pass through the ribs (141). The clamp (14) securely holds the needle hub (20) in place after the needle (21) passes through the passage (12) and the needle hub (20) passes through the ribs (141). The clamp (14) can prevent the safety cover from detaching from the syringe.

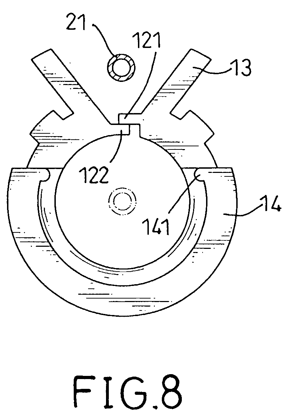

With reference to Fig. 8, in a third embodiment of the safety cover for a syringe in accordance with the present invention, the overall width of the passage is greater than the diameter of the needle (21). However, the passage includes an upper plate (121) formed at the bottom of one sidewall overlapping a lower plate (122) formed at the bottom of the other sidewall. The plates (121, 122) extend horizontally from the corresponding sidewall of the passage. The upper plate (121) overlaps the lower plate (122) to close the passage to prevent germs or viruses on the used needle from spreading via air. The first and second plates (121, 122) are flexible to allow the needle to pass between the plates (121, 122).

The safety cover for a syringe in accordance with the present invention can prevent the medical worker from accidentally pricking his finger while covering the needle of the syringe and reduce the probability of accidental infection. Furthermore, the safety cover for a syringe has several advantages.
1. The present invention is a single-piece cover that can achieve the goal of preventing the medical worker from accidental infection without making the safety cover unnecessarily complex.
2. The price of the safety cover for syringe is low in that it can be used with a conventional syringe available in the market. The manufacturer does not need to change the syringe to accommodate the safety cover. Consequently, there is no additional cost to pass along to the consumer.
3. The cover attaches to a conventional syringe without modifying the syringe. Consequently, the potential functions of the syringe are unchanged.

Although the invention has been explained in relation to its preferred embodiment, it is to be understood that many other possible modifications and variations can be made without departing from the spirit and scope of the invention as hereinafter claimed.

## Claims

1. A safety cover for a syringe including a needle hub attached to one end of the syringe and a needle securely attached to said needle hub, said safety cover comprising:
a tubular body containing a receiving space and having a first end, a second end and a longitudinal slot defined in an outer periphery from said first end and extending towards said second end, said slot having a passage defined in a bottom defining said slot to communicate with said receiving space inside said tubular body, wherein said needle can pass through said passage and into said receiving space of said tubular body.

2. The safety cover for a syringe as claimed in claim 1, wherein said slot has a length longer than that of said needle.

3. The safety cover for a syringe as claimed in claim 1, wherein said slot has a V-shaped cross section.

4. The safety cover for a syringe as claimed in claim 1, wherein said first end of said tubular body includes a cutout defined to form a C-shaped clamp using said passage as a center line, said clamp including two ribs respectively formed on opposed distal edges of said clamp and a distance between said two ribs being so configured that it is slightly less than a diameter of said needle hub.

5. The safety cover for syringe as claimed in claim 1, wherein said slot includes two sidewalls and said tubular body includes two wings respectively extending out from each sidewall at an angle of said sidewall of said slot to form a funnel-shape.

6. The safety cover for a syringe as claimed in claim 1, wherein said passage has a width greater than a diameter of said needle, said passage including an upper plate extending from a bottom of one sidewall overlapping a lower plate extending horizontally from a bottom of the other sidewall, said upper plate and lower plate respectively extending horizontally from said sidewalls of said passage to close said passage.

7. The safety cover for a syringe as claimed in claim 4, wherein said passage has a width greater than a diameter of said needle, said passage including an upper plate extending horizontally from a bottom of one sidewall overlapping a lower plate extending horizontally from a bottom of the other sidewall, said first plate and second plate respectively extending horizontally from said sidewalls of said passage to close said passage.
